# EUROPEAN PATENT APPLICATION

(11) **EP 1 043 407 A2**
(43) Date of publication of application: **11.10.2000**
(21) Application number: 00107333.7
(22) Date of filing: 04.04.2000
(51) Int. Cl.: C12Q 1/70

(54) **Nucleotide sequence for detection and characterization of the HIV-1 field isolates participating in the recombination processes directed by the chi sequence**

(30) Priority: 09.04.1999 IT MI990729
(71) Applicant: Diapharm Limited, St. Peter Port, Guernsey, Channel Islands GY1 4EJ (GB)
(72) Inventor: Veljkovic, Veljko, 11000 Belgrad (YU); Veljkovic, Nevena, 11000 Belgrad (YU); Prljic, Jelena, 11000 Belgrad (YU); Metlas, Radmila, 11000 Belgrad (YU)
(74) Representative: Minoja, Fabrizio, Dr.

(57) **Abstract**

Oligonucleotides for use as primers in polymerase chain reactions for diagnostic purposes, said oligonucleotides having 110 to 40 nucleotide bases and comprising the sequence **GCTGGTGG** inserted between highly conserved nucleotide sequences corresponding to the domain of HIV-1 gp120 gene coding the second conserved region.

SEQUENCE LISTING

<110> Diapharm Limited

<120> Nucleotide sequence for detection and characterisation
      of the HIV-1 field isolates participating in the
      recombination processes directed by the Chi sequence

<130> diapharm

<140>
<141>

<160> 5

<170> PatentIn Ver. 2.1

<210> 1
<211> 21
<212> DNA
<213> Human immunodeficiency virus type 1

<400> 1
atagtacagc tggtggactc t                                           21


<210> 2
<211> 11
<212> DNA
<213> Human immunodeficiency virus type 1

<400> 2
cagctggtgg a                                                      11


<210> 3
<211> 21
<212> DNA
<213> Human immunodeficiency virus type 1

<400> 3
ttggagagca atggctagtg a                                           21


<210> 4
<211> 20
<212> DNA
<213> Human immunodeficiency virus type 1

<400> 4
atttctgggt cccctcctga                                             20


<210> 5
<211> 243
<212> DNA
<213> Human immunodeficiency virus type 1

<400> 5
agtacagctg gtggactctc cttgtccggg ttctcgctct caaggaacag cagctgcgca 60
aaccaggttg gcagtctggt cattgacctc acccaccagg aagatcacgc gctccttcaa 120
cacgcgaata aatgtcgtag gaccgctcgc cgcggccgga ctgctcaatg accatgggga 180
tcacaagggc ttgcgtttcc aatgcgctca tacgtttctc cagtcaggag gggacccaga 240
aat 243

## Description

### THE FIELD OF THE INVENTION

The present invention is directed to nucleotide sequences of retroviral genomes of chimeric viruses representing the natural recombinants between HIV and coinfected pathogens and their use for detection of the chimeric HIV-1 field isolates in human sera.

### BACKGROUND OF THE INVENTION

The HIV-1 envelope glycoprotein gp120 represents the main antigen important for protection against the virus or infected cells, by both humoral and cell-mediated response. In spite of considerable sequence divergence among different HIV-1 isolates, the gp120 molecules have retained their binding tropism for the CD4 receptor, as well as the ability to induce cytopatic effects in vitro. For this reason the identification of HIV-1 gp120 domains that are responsible for interaction with CD4 molecule seems fundamental to obtaining a core protein with binding activity, that may prevent the AIDS virus from entering cells.

In order to identify the domain of HIV-1 gp120 that is responsible for its recognition and targeting to the CD4 receptor, the informational spectrum method (ISM) [Veljkovic et al., IEEE Trans. Biomed. Eng., 32:337 (1985); Veljkovic and Cosic, Cancer Biochem. Biophys., 9:139 (1987)] was used to identify possible sequences. It has been identified the nanopeptide NAKTIIVQL (denoted as "peptide V"), belonging to the C-terminus of the second conserved region of the gp120 molecule, as being critical for gp120/CD4 recognition and subsequent binding [Veljkovic and Metlas, Cancer Biochem. Biophys., 10:206 (1988)].

Further investigation of the domain encompassing "peptide V", revealed the sequence motif VQL (N/V) ES common to HIV-1 gp120 and the first framework region (FR1) of the human IgG heavy chain variable region (sbgroup III (VhIII) [Veljkovic and Metlas, Immunol. Lett., 26:191 (1990); Metlas et al., Biochem. Biophys. Res. Commun., 179:1056 (1991)]. The alternation of well-conserved and variable regions within the VhIII family of genes has led other authors to suggest that the constant regions, including the motif VQL(N/V)ES, might carry noncoding functions involved in rearrangement processes [Kenter and Birshtein, Nature, 293:402 (1981); Tuter and Riblet, Proc. Natl. Acad. Sci. USA, 86:7460 (1989)]. Previously, it has been reported that the nucleotide sequence GCTGGTGG, encoding the amino acids 2 - 3 (QLV) of this motif in VhIII, represents the Chi signal promoting generalized recombination in prokaryotes [Tuter and Riblet, Proc. Natl. Acad. Sci. USA, 86:7460 (1989)]. It is assumed that the role of CHI at this position in the Ig gene is to promote preferentially VhIIIgene expression during fetal B cell development [Tuter and Riblet, Proc. Natl. Acad. Sci. USA, 86:7460 (1989)].

Downstream from the putative Chi sequence, a heptamer sequence, CAGTCTG, has been identified. Five of the seven bases in the sequence match the consensus recombination signal CACTGTG already found to be involved in V-(D)-J recombination of the Ig gene (reviewed in Tonegava S., Nature, 302:575 (1983)]. A similar sequence exists at the same position in the gp120 gene of all HIV-1 isolates.

Within the VhIII gene, 26 nucleotides downstream from the Chi sequence, the second IgH specific recombination signal, TACTGTG, have been localized [Tuter and Riblet, Proc. Natl. Acad. Sci. USA, 86:7460 (1989)]. The HIV-1 gp120 gene, downstream of the Chi-like sequence at a similar distance (260 - 270 nucleotides, depending on the HIV-1 isolate), contains the homologous sequence TACTGTA, representing a putative recombination signal. This sequence is conserved in all HIV-1 isolates.

Both of the above recombination signals have been shown to take part in the rearrangement process in heterologous systems [Tuter and Riblet, Proc. Natl. Acad. Sci. USA, 86:7460 (1989)]. It has been shown experimentally that a similar level of conservation (one mismatch at the 3' end) can be sufficient to elicit a recombination event [Kenter and Birshtein, Nature, 293:402 (1981)].

The nucleotide sequence between these two putative recombination signals encodes the third hypervariable domain (V3), representing the dominant but strain-specific neutralizing epitope of HIV-1 gp120 [Rusche et al., Proc. Natl. Acad. Sci. USA, 85:3198 (1988)].

Based on the above facts it was hypothesized that (1) the HIV-1 gp120 gene is inherently recombinogenic allowing to some viral subclones to evolve toward complete and active Chi sequence within the second conserved domain, and (2) under this condition gene region coding for V3 loop, representing functionally and immunologically important region of HIV-1 gp120, may be involved in homologous recombination.

### SUMMARY OF THE INVENTION

The present invention is directed to a primer for detection by RT-PCR (reverse-transcriptase - polymerase chain reaction) of HIV-1 subclones carrying the Chi recombination sequence, GCTGGTGG, in the gene coding for envelope glycoprotein gp120.

More particularly, the invention is directed to oligonucleotides for use as primers in polymeras chain reactions for diagnostic purposes, said oligonucleotides having 10 to 40 nucleotide bases and comprising the GCTGGTGG nucleotide sequence inserted between highly conserved nucleotide sequences corresponding to the domain of HIV-1 gp120 gene coding the second conserved region.

The invention is further directed to a diagnostic method for detection of HIV-1 isolates, which comprises subjecting the serum from a patient to a polymerase chain reaction (RT-PCR) using an oligonucleotide of the invention as the first primer and a sequence derived from the third conserved region of gp120 as the second primer.

### DETAILED DISCLOSURE OF THE INVENTION

The oligonucleotides of the invention can be prepared according to conventional methods well-known to those skilled in the art.

A preferred oligonucleotide has the following sequence **ATAGTACAGCTGGTGGACTCT** (Sequence Id N° 1) comprising of the sequences ATAGTA and CTCT derived from the gp120 gene domain coding the second conserved region, and the consensus sequence **CAGCTGGTGGA** (Sequence Id N° 2) derived from the human VhIII gene domain coding the FR1 region.

The oligonucleotides of the invention, in combination with any oligonucleotide representing the conserved sequence derived from the gp120 gene downstream from the end of the third hypervariable region, could be used for detection in sera as well as genetic material of HIV patients the HIV-1 subclones, carrying the Chi sequence and participating in recombination with the host genes and recombination with the coinfected viral and bacterial pathogens.

The oligonucleotides of the invention can be used as important markers for monitoring of disease progression, as well as effects of the therapeutic treatment of HIV patients.

The invention is based on the finding, in the sera of HIV patients, of HIV-1 subclones that could participate in recombination directed by the Chi recombination signal. In fact, by using as primer in an RT-PCR an oligonucleotide of the invention and an oligonucleotide deriving from the highly conserved sequence of the HIV-1 gp120 third conserved domain, an amplification product was obtained which does not belong to the HIV-1 gp120. Analysis of this fragment showed its high homology, on both the protein and nucleotide level, to the clp protease from the *Hemophilus influenzae*. Taking into account that (1) *H*. *influenzae* represents a very recombinogenic organism containing in its genome an unusual number of the Chi-like sequences, and (2) that this pathogen is usually coinfected with HIV-1, it can be concluded that the detected subclone of HIV-1 possesses in its gp120 gene an active Chi recombination promoter, representing a chimeric virus that acquired a gene fragment from *H*. *influenzae*.

It has been hypothesized that the sequence homology between the HIV-1 gp120 and human Ig genes indicates the acquisition of host genes by the virus. This possible step in HIV-1 evolution represents the potential base for the escape mutant generation. The Chi sequence in HIV-1 gp120 gene may be involved in the promotion of chromosomal rearrangement and the formation of the aberrant immunoglobulins, leading to inadequate humoral and cell-mediated immune response. The process of such abnormal recombination may also increase the frequency of B cell malignancies, which are observed in HIV patients. Besides these hypothesized events, Chi sequence also could promote recombination between the HIV-1 gp120 and the coinfected viral and bacterial pathogens, resulting in generation of the chimeric viral subclone which could additionally influence the host immune system (i.e. as superantigen) and escape neutralization by antibodies. The verification of these hypotheses does not affect in any way the validity of the present invention, which will be now illustrated in detail in the following examples.

### EXAMPLE 1

### Amplification by RT-PCR of the gene sequence of HIV-1 gp120 carrying Chi sequence oligonucleotide of primers

The primer of the invention, in the following referred to as P2, having the sequence **ATAGTACAGCTGGTGGACTCT** (Sequence Id N° 1), in combination with primer P1, having the sequence **TTGGAGAGCAATGGCTAGTGA** (Sequence Id N° 3), derived from the consensus nucleotide sequence from the HIV-1 gag gene, or the primer P3, having the sequence **ATTTCTGGGTCCCCTCCTGA** (Sequence Id N° 4) derived from the gp120 gene domain coding the third conserved region have been used.

The study was performed on plasma samples of 11 HIV-1 infected patients and 3 blood donors as negative controls. Plasma samples were stored frozen at -80°C. The samples were subjected to a maximum of two freeze-thaw cycles. All samples were heat inactivated at 56°C for 30 min before further analysis.

### RNA Isolation

Total RNA was extracted from 200 µl of plasma by using a RNA fast isolation system (Biotech Lab., USA) according to the manufacturer's protocol, purified by O/N isopropanol precipitation at -20°C and ethanol washes. RNA pellet was resuspended in 6 µl milliQ water.

### Reverse transcriptase assay

cDNA was obtained from 2h RT reaction at 42°C by using the antisense primer P3. The reaction was performed in a volume of 40 µl and the reaction conditions were as follows: 2.5 µl RNA and 17.5 µl milliQ water were heated at 95°C for 3 min. Then were added 20 U RNasin (Promega, USA), 4 µl AMV Reverse Transcriptase 5X Reaction Buffer (Promega, USA), 10 pmol each of dNTPs (Promega, USA), 30 pmol primer P3 and 20 U AMV Reverse Transcriptase (Promega, USA).

### PCR Amplification

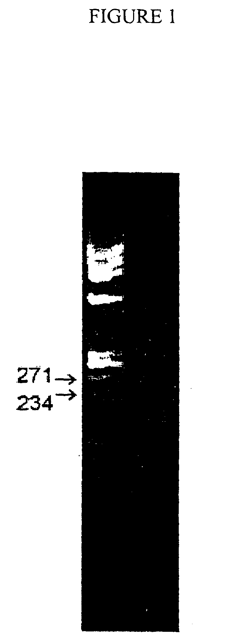

cDNA were analyzed by Seminested PCR. First round outer primers were: P1 and P3. The second round primers were: outer primer P3 and inner primer P2. First PCR reaction was performed in a 50 µl reaction mixture containing 3 µl of RT reaction product, Expand HF buffer 10X conc. with 15 mM MgCl₂ (Boehringer Mannheim GmbH.), 2.6 U Expand TM High Fidelity enzyme mix (Boehringer Mannheim GmbH.), 300 µmol dNTPs, 30 pmol of outer primers. The amplification profile was as follows: 93°C for 5 min, 40 cycles of 80 s at 93°C, 80 s at 55°C, 3 min at 68°C and 68° C for 10 min. 1 µl of diluted product of first round PCR (diluted 1:100) was amplified in a Seminested PCR. Other components of the PCR reaction mixture were: Taq DNA Polymerase 10X Buffer (Promega, U.S.A.), 1.5 mM MgCl₂, 2.5 U Taq DNA polymerase (Promega U.S.A.), 200 µmol dNTPs, 30 pmol of each primer. The amplification profile was as follows: 95°C for 5 min, 40 cycles of 40 s at 95°C, 40 s at 55°C, 70 s at 72°C. The last extension was performed at 72°C for 10 min, Total volume of reaction was 50 µl. All amplification reactions were done in a RoboCycler gradient 40 (Stratagene). Amplified products were detected in 1.5 hr, 88 V, 3% agarose gel electrophoresis and stained with ethidium bromide (Fig 1.).

### EXAMPLE 2

### Cloning and sequencing of the RT-PCR product

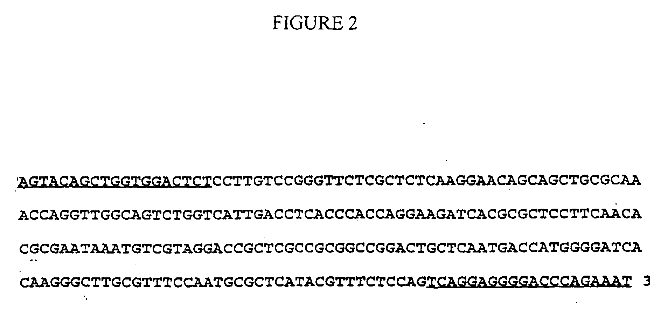

The PCR products were purified with the QIAEX II Agarose Gel Extraction kit according to the manufacturer's protocol. PCR-purified product was cloned in pGEM- T Vector. Plasmid DNA was extracted by means of the WizardTM Plus minipreps DNA Purification System (Promega). The presence and the size of the insert in the plasmid clones were verified by restriction enzyme analysis. Clones were sequenced on both stands, and the obtained nucleotide sequence is given in Fig. 2.

### EXAMPLE 3

### Sequence analysis

For analysis of the sequence obtained by the cloning and subsequent sequencing of the RT-PCR product covered with the primers P3 and P4 the program TBLASTX [Gish, W. (1994). unpublished. Altschul, Stephen F., Warren Gish, Webb Miller, Eugene W. Myers, and David J. Lipman (1990). Basic local alignment search tool. J. Mol. Biol. 215:403-10. was used. This program was used for comparison of the cloned sequence versus EMBL nucleic acid sequence database (Release 52) encompassing 351,452 sequences; 478,269,690 total letters.

The product proved to have a large degree of homology to the H132754 sequence from *Haemophilus Influenzae*, corresponding to the bases 756687 to 766871 of the complete genoma.

## Claims

1. Oligonucleotides for use as primers in polymerase chain reactions for diagnostic purposes, said oligonucleotides having 10 to 40 nucleotide bases and comprising the sequence **GCTGGTGG** inserted between highly conserved nucleotide sequences corresponding to the domain of HIV-1 gp120 gene coding the second conserved region.

2. Oligonucleotide as claimed in claim 1, having the sequence **ATAGTACAGCTGGTGGACTCT** (Sequence Id N° 1).

3. A diagnostic method for the determination of HIV-1 isolates, which comprises subjecting the serum from a patient to a polymerase chain reaction (RT-PCR) using an oligonucleotide as claimed in claims 1-2 as the first primer and a sequence derived from the third conserved region of gp120 as the second primer.
